# FASCICULE DE BREVET EUROPEEN

(11) **EP 1 865 855 B1**
(45) Date de publication et mention de la délivrance du brevet: **27.08.2008**
(21) Numéro de dépôt: 06726311.1
(22) Date de dépôt: 06.04.2006
(51) Int. Cl.: A61B 17/072

(54) **SYSTEME DE MISE EN PLACE DE MOYENS FORMANT RENFORT D'UNE SUTURE**
SYSTEM ZUM ANBRINGEN VON MITTELN WELCHE EINE NAHT VERSTÄRKEN
PLACEMENT SYSTEM FOR A MEANS WHICH REINFORCES A SUTURE

(30) Priorité: 07.04.2005 FR 0503463
(43) Date de publication de la demande: 19.12.2007
(73) Titulaire: SOFRADIM PRODUCTION, 01600 Trévoux (FR)
(72) Inventeur: SGRO, Jean-Claude, 21000 Dijon (FR)
(74) Mandataire: Cardy, Sophie Marie
(86) Numéro de dépôt international: PCT/FR2006/050305
(87) Numéro de publication internationale: WO 2006/106269

(56) Documents cités:
- WO-A-99/02090
- DE-A1- 19 924 311
- US-A- 5 575 803
- US-A- 6 099 551
- US-A1- 2002 151 911

## Description

L'invention concerne un système de mise en place de moyens formant renfort d'une suture.

Dans le cadre d'une intervention chirurgicale, où il s'agit de sectionner au moins une partie d'un organe, le praticien est amené à suturer la partie de l'organe qui reste dans le corps du patient, et éventuellement l'autre partie (qui est à retirer ou à déplacer). Pour ce faire, le praticien utilise généralement une pince à suture ; les pinces à suture de type connu sont généralement des agrafeuses linéaires coupantes qui possèdent une lame ou un couteau et qui sont rechargeables avec des recharges pour permettre plusieurs agrafages pendant le déroulement de l'opération. Les types de recharges peuvent éventuellement être différents, en particulier pour avoir des agrafes de tailles différentes d'une recharge à l'autre.

La mise en place de moyens formant renfort d'une suture, permet de renforcer la zone sectionnée et qui risque de s'endommager, notamment de se déchirer sous l'action d'un agrafage, en particulier dans le cas d'un intestin distendu ou inflammatoire, ou de ne pas être étanche, en particulier dans le cas de tissus pulmonaires.

Dans les dispositifs de renfort connus, un manchon de tissu, résorbable ou non, entoure les extrémités d'une pince à suture automatique, de sorte qu'au moment où les agrafes sont posées, le tissu est pris par les agrafes et empêche celles-ci de s'enfoncer dans la zone à traiter.

Le document WO-A-99/02090 décrit un système selon le préambule de la première revendication.

Cependant, ces manchons de renfort sont difficilement maintenus en place sur les branches de la pince.

L'invention a pour but de fournir un système de mise en place de moyens formant renfort qui permet de maintenir le renfort sur la pince à suture avant la section de l'organe à traiter, et qui permet aux moyens formant renfort d'être pris par les moyens formant sutures au moment où les moyens formant sutures sont posés.

Ce but de l'invention est atteint par le fait que le système comporte des moyens formant renfort pour renforcer une zone destinée à être suturée, comprenant une première partie de renfort et une deuxième partie de renfort, une portion d'attrapage reliant lesdites première et deuxième parties des moyens formant renfort et permettant d'attraper la zone à suturer, et des moyens de passage des deux branches d'une pince à suture, tels que ladite première partie des moyens formant renfort soit apte à être en regard de l'une des deux branches et ladite seconde partie des moyens formant renfort soit apte à être en regard de l'autre des deux branches, ledit système de mise en place comportant en outre des moyens de préhension formés par deux bandes reliées aux moyens formant renfort qui s'étendent vers l'arrière en dehors de la zone des branches.

Ainsi, les moyens formant renfort sont retenus sur la pince à suture avant la section par les moyens de passage ; pendant la section et la mise en place des moyens formant sutures, les moyens de renfort sont suturés sur la partie d'organe sectionnée ; et, en fin d'opération, après avoir séparé les moyens de préhension, les moyens de renfort restent en place.

En outre, la portion d'attrapage permet d'attraper et de maintenir l'organe à sectionner en l'attirant vers la pince à suture, ladite portion d'attrapage permettant d'attraper la zone à suturer et éventuellement de l'attirer à l'extérieur du corps du patient à opérer.

Egalement, les moyens de préhension facilitent la mise en place de la pince à suture dans le système, et en particulier dans les moyens de passage : ces moyens formant préhension sont de préférence d'une longueur suffisante pour que le praticien puisse les attraper avec la main ou une pince.

Selon une première forme de réalisation, lesdits moyens de passage sont deux ouvertures de contour fermé situées respectivement sur lesdites première et deuxième parties des moyens formant renfort et/ou les moyens de préhension, à l'écart de la portion d'attrapage.

Selon une deuxième forme de réalisation, le système comporte en outre des moyens formant support et des moyens d'assemblage détachables permettant d'assembler les moyens formant renfort et les moyens formant support pour délimiter lesdits moyens de passage formés de deux logements destinés à loger les deux branches d'une pince à suture. Avec les moyens formant support, la première partie de renfort forme l'un des deux logements, tandis que la seconde partie de renfort forme l'autre des deux logements.

Ainsi, les moyens formant renfort se situent de part et d'autre de l'organe à sectionner. Il s'ensuit que lorsque les deux branches de la pince à suture, logées dans lesdits logements, viennent enserrer l'organe à sectionner et à suturer, les deux moyens formant renforts se trouvent en regard de ce dernier, ainsi pris en sandwich, permettant ainsi de renforcer toute la zone suturée.

De manière connue, lorsque l'organe est sectionné en deux, les deux parties d'organe sont suturées. On comprend que les moyens formant renfort selon l'invention, se situent après section, sur chacune des deux parties d'organe sectionnées et suturées.

Dans ce cas, on comprend que les moyens de préhension facilitent le maintien des logements en regard de la partie active de la pince et le retrait des moyens formant support, après mise en place des moyens formant renfort sur la zone suturée et renforcée.

Ainsi, après section et suture de la zone à traiter, les moyens formant support de chacun des deux logements peuvent être retirés.

Afin de protéger cette zone suturée et renforcée, il peut être prévu de recouvrir les moyens formant sutures mis en place par la pince. Pour ce faire, le système comporte préférentiellement des moyens formant maintien permettant, après suture de la zone à suturer, de maintenir ensemble les première et deuxième parties de renfort.

En fait, les première et seconde parties de renfort sont maintenues d'une part, contre l'organe sectionné par l'intermédiaire des moyens formant sutures et d'autre part, ensemble l'une contre l'autre en dehors de la zone suturée et renforcée, après avoir été repliées par dessus cette dernière.

Ainsi, l'utilisation de ces moyens formant renfort permet en outre de protéger et d'empêcher les adhérences qui s'organisent autour de la partie d'organe restée en place, appelée moignon, afin de pouvoir retrouver facilement cette dernière lors d'une ré-intervention, qui intervient généralement trois à cinq semaines plus tard. En effet, par exemple dans le cas d'une résection du sigmoïde sans rétablissement de continuité, si le moignon n'a pas été protégé, les adhérences s'organisent autour de lui, en particulier un magma d'anses intestinales grêles collées, se constitue. Lors de la ré-intervention, il faut alors disséquer les anses grêles, au risque septique de les percer. Par ailleurs, parfois un rétablissement de continuité peut être réalisé sous coelioscopie ; dans ce cas, s'il y a trop d'adhérences, il faut abandonner cette voie et reprendre la voie ouverte, moins intéressante pour le patient. Ainsi, l'adjonction de moyens formant renfort peut prévenir la formation d'adhérences sur le moignon évitant les infections locales et les adhérences.

Les moyens formant maintien peuvent être de type connu, comme par exemple des moyens de collage ou des moyens d'attache du type autoagrippant (par exemple Velcro^{®}), etc.

Avantageusement, les moyens formant maintien comportent des premiers éléments de maintien disposés sur la première partie de renfort et des seconds éléments de maintien disposés sur la seconde partie de renfort, lesdits premiers et seconds éléments de maintien étant aptes à coopérer pour maintenir lesdites première et seconde parties de renfort l'une contre l'autre.

Afin d'éviter d'entraîner d'autres organes avec les moyens formant maintien au moment où les moyens formant renfort sont mis en place autour de l'organe à sectionner, il peut être prévu que les moyens formant renfort se présentent sous la forme d'une bande présentant une première face et une seconde face, ladite première face étant tournée vers l'intérieur des logements respectifs lorsque les moyens formant support et les moyens formant renfort sont assemblés, que les premier et second éléments de maintien sont disposés sur ladite première face, et qu'après séparation des moyens formant support et des moyens formant renfort, les première et seconde parties de renfort sont aptes à être repliées sur elles-mêmes de sorte que les premier et second éléments de maintien puissent s'engager les uns avec les autres pour maintenir lesdites première et seconde parties ensemble.

L'invention sera bien comprise et ses avantages apparaîtront mieux à la lecture de la description détaillée qui suit, de modes de réalisation de l'invention représentés à titre d'exemple non limitatifs. La description se réfère aux dessins annexés sur lesquels :
- la figure 1 représente une vue en perspective du système selon l'invention selon une première forme de réalisation,
- les figures 2A à 2C représentent une vue en perspective du système de la figure 1 pendant son utilisation,
- la figure 3 représente une vue en perspective du système selon une deuxième forme de réalisation,
- la figure 4 représente une vue en perspective de la mise en place préalable du système de la figure 3 autour de l'organe à opérer,
- la figure 5 représente une vue schématique en perspective de zones suturées et renforcées,
- la figure 6 représente une vue schématique en perspective d'une zone suturée et renforcée après recouvrement,
- la figure 7 est une vue en perspective du système selon une variante de réalisation ; et
- la figure 8 est une vue en perspective du système selon une autre variante de réalisation.

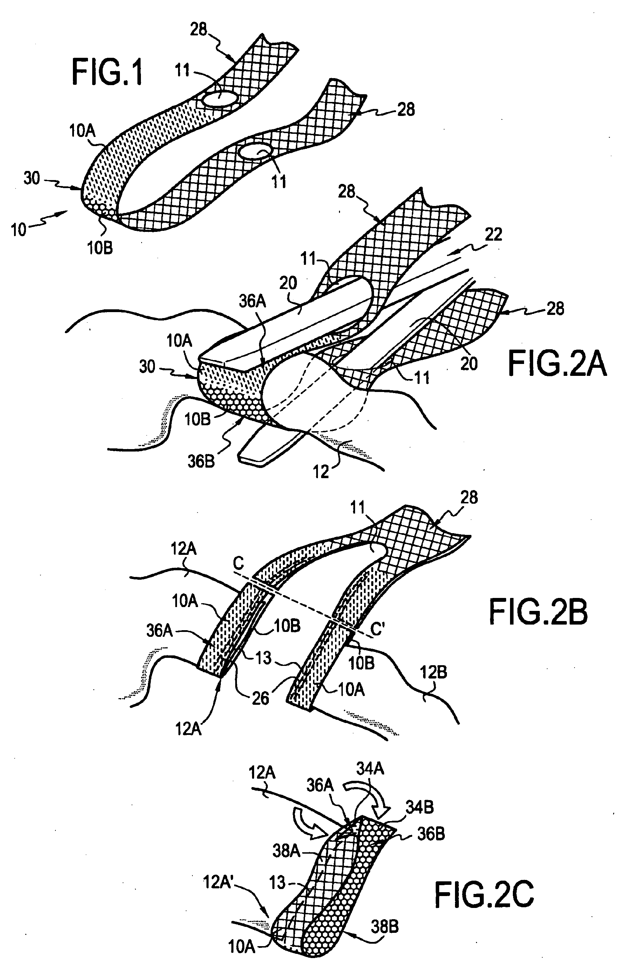

Les figures 1 et 2A représentent un système de mise en place de moyens formant renfort d'une suture selon une première forme de réalisation qui comporte des moyens formant renfort 10 destinés à renforcer au moins une zone 12 qui va être sectionnée et suturée.

Ces moyens formant renfort 10 sont composés d'une première partie de renfort 10A et d'une deuxième partie de renfort 10B, reliées entre elles par une portion d'attrapage 30 permettant d'attraper et de retenir la zone 12 de l'organe, en jouant un rôle de lacette.

Soit les première et deuxième parties de renfort 10A et 10B font partie de la même bande, soit elles sont attachées ensemble, par exemple par une couture, directement l'une à l'autre, ou par l'intermédiaire d'une autre pièce appartenant à la portion d'attrapage 30.

Les extrémités des première et deuxième parties de renfort 10A et 10B opposées à la portion d'attrapage 30 se prolongent par des moyens de préhension 28, ce qui permet de faciliter la préhension des moyens formant renfort 10 et le maintien de l'ensemble du système sur les branches 20 de la pince à suture 22. En l'espèce, les moyens de préhension 28 sont formés par deux bandes qui sont chacune formées par la même ou une autre bande que celle formant les première et deuxième parties de renfort 10A et 10B.

Ainsi, les moyens de préhension 28 sont reliés aux moyens formant renfort (10A et 10B) et s'étendent en dehors de la zone des branches 20 (à droite sur la figure 2A), afin de pouvoir être attrapés par la main ou la pince du chirurgien effectuant l'opération ou de son assistant.

Comme il apparaît sur la figure 2A, après avoir enserré la zone 12 de l'organe, on introduit les branches 20 de la pince 22 dans des moyens de passage qui sont dans ce cas constitués de deux ouvertures 11 de contour fermé (circulaire sur les dessins) situées à distance de la portion d'attrapage 30, soit dans les première et deuxième parties de renfort 10A et 10B, soit dans les portions de bande formant les moyens de préhension 28 (situation illustrée), soit à cheval sur les moyens formant renfort 10 et les moyens de préhension 28.

De cette façon, après avoir entouré la zone 12 de l'organe, on peut insérer chaque branche 20 de la pince dans une ouverture 11, la face active de chaque branche 20 faisant face respectivement à la première et la deuxième parties de renfort 10A et 10B (voir figure 2A), de part et d'autre de la zone 12 de l'organe.

Au moment de l'utilisation de la pince à suture 22, les deux branches 20 sont rapprochées l'une de l'autre de part et d'autre de l'organe 12 à opérer. Ce dernier est alors coupé en deux portions 12A et 12B (voir figure 2B) qui sont simultanément suturées, en l'espèce agrafées à l'aide des agrafes 26 stockées dans les branches 20 de la pince à suture 22. Lors de la section de l'organe 12, les première et seconde parties de renfort 10A et 10B, ainsi que la portion d'attrapage 30, sont, elles aussi, coupées en deux bandelettes ou lanières sensiblement identiques jusqu'à l'ouverture 11, dans le sens longitudinal, parallèle au sens longitudinal des branches 20 de la pince à suture 22.

Ainsi, une lanière de la première partie de renfort 10A et une lanière de la deuxième partie de renfort 10B sont à présent assemblées à la partie 12A au niveau d'une suture 13, par l'intermédiaire des agrafes 26, afin de former la partie 12A' qui correspond à la partie 12A qui a été suturée et renforcée.

Les parties 12A et 12B sont analogues, de sorte que la suite de la description n'est faite que pour la portion 12A, en relation avec les figures 2B et 2C.

Comme il apparaît sur la figure 2B, on réalise une découpe, par exemple aux ciseaux, au niveau de la ligne C-C', afin de séparer les deux sutures 13 des moyens de préhension 28. Ainsi, la zone suturée et renforcée 12A' est séparée des moyens de préhension 28 mais aussi de l'autre portion 12B de l'organe.

Ensuite, comme il apparaît sur la figure 2C, on retourne (voir les flèches) les première et deuxième parties de renfort 10A, 10B l'une sur l'autre et sur la suture 13 qui est ainsi protégée.

Plus précisément, les première et deuxième parties de renfort 10A, 10B sont assemblées grâce à des moyens formant maintien 34 aptes à coopérer ensemble pour maintenir l'une contre l'autre les première et seconde parties de renfort 10A et 10B.

En l'espèce, les moyens formant maintien 34 comportent des premiers éléments de maintien 34A disposés sur la première partie de renfort 10A et des seconds éléments de maintien 34B disposés sur la seconde partie de renfort 10B.

Les premiers (seconds) éléments de maintien 34A (34B) sont disposés sur une face 36A (36B) de la première (deuxième) partie de renfort 10A (10B), qui, lorsque cette dernière était reliée aux moyens de préhension 28, était tournée en direction opposée par rapport à l'organe 12.

En fait, cette face 36A est tournée vers les moyens formant sutures (lame et agrafes 26 de la pince 22), avant la mise en place sur la zone 12 à sectionner et suturer de sorte de ne pas s'accrocher pendant la mise en place du système autour de l'organe. En outre, dans cette configuration, les premiers éléments de maintien 34A étant, au début de l'opération, tournés en direction opposée par rapport aux seconds éléments de maintien 34B, ils ne peuvent pas jouer leur rôle de liaison à ce moment-là, puisqu'ils ne peuvent pas coopérer l'un avec l'autre.

Sur la figure 2C, on voit donc qu'après retournement des première et seconde parties de renfort 10A et 10B, leurs faces 36A et 36B portant respectivement les premiers éléments de maintien 34A et les seconds éléments de maintien 34B, se retrouvent face à face de sorte que les moyens formant maintien 34 jouent leur rôle et réalisent l'assemblage. Dans cette position, c'est l'autre face 38A, 38B des première et seconde parties de renfort 10A et 10B qui se retrouve tournée vers l'extérieur.

A titre d'exemple, les moyens formant maintien 34 sont du type autoagrippants, avec les premiers éléments de maintien 34A formés d'éléments en forme de crochet ou de champignon et les seconds éléments de maintien 34B formés d'éléments en forme de boucle.

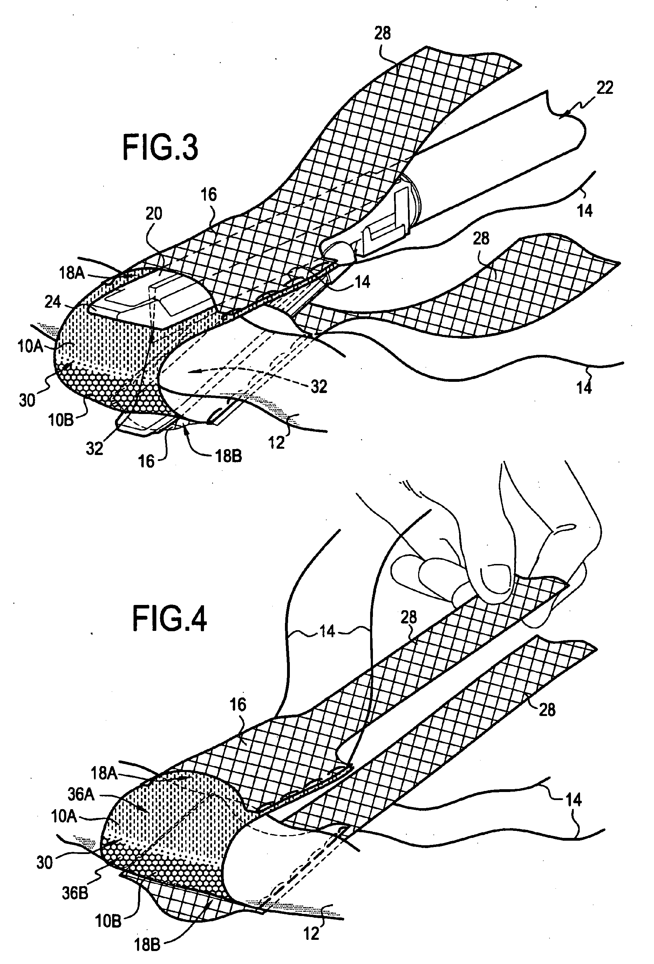

On se reporte maintenant aux figures 3 et 4 représentant un système selon une deuxième forme de réalisation de l'invention.

Dans ce cas, les moyens formant renfort 10 sont assemblés, à l'aide de moyens d'assemblage 14 détachables, à des moyens formant support 16 (comprenant deux bandes) pour former deux logements 18A, 18B dans lesquels chacune des deux branches 20 d'une pince à suture 22 du type connu peut être disposée. Afin de s'assurer que les moyens de renfort seront bien disposés le long de toute la zone suturée, les logements 18A et 18B sont préférentiellement traversants, c'est-à-dire débouchants à leurs deux extrémités ; ainsi, l'extrémité libre 24 de la branche 20 de la pince à suture 22 peut dépasser hors des moyens formant renfort 10 et au-delà de la limite de la zone 12.

Les moyens formant renfort 10 sont disposés en regard de la zone active de la branche 20 de la pince à suture 22, en l'espèce en face des moyens formant sutures, comportant par exemple, d'une part, des agrafes 26 stockées dans l'une des deux branches 20 et, d'autre part, des moyens de repli des agrafes, formés dans l'autre des deux branches 20 qui comporte aussi la lame.

Comme dans le cas de la première forme de réalisation, les moyens formant renfort 10 comportent une première partie de renfort 10A et une seconde partie de renfort 10B, la première partie de renfort formant avec les moyens formant support 16 l'un des deux logements 18A, tandis que la seconde partie de renfort 10B forme avec les moyens formant support 16 l'autre des deux logements 18B.

Afin de faciliter la préhension des moyens formant support 16 et le maintien de l'ensemble du système sur les branches 20 de la pince à suture 22, des moyens de préhension 28 sont reliés à et prolongent les moyens formant support 16.

En l'espèce, les moyens de préhension 28 sont formés par une bande qui s'étend vers l'arrière (à droite sur les figures) en dehors de la zone des branches 20, afin de pouvoir être attrapée par la main ou la pince du chirurgien effectuant l'opération ou de son assistant.

De même, les moyens d'assemblage 14, en l'espèce un fil 14 qui permet d'assembler les moyens formant renfort 10 et les moyens formant support 16 le long de leurs bords longitudinaux, dépasse de préférence au-delà du logement 18 au voisinage des moyens de préhension 28, de sorte qu'il puisse être rapidement et facilement attrapé après l'opération de section et de suture.

Les deux logements 18A et 18B, illustrés sur la figure 3 sont assemblés, par l'intermédiaire d'une portion d'attrapage 30 qui permet d'attraper l'organe à traiter et de le rapprocher vers la pince à suture 22.

En outre, afin de renforcer les deux zones qui sont sectionnées et suturées simultanément à l'aide de la pince à suture 22, il peut être prévu deux fils d'assemblage 14 par logement 18A, 18B, comme illustré sur la figure 3.

Avant opération, comme illustré sur la figure 4, le système est utilisé pour attraper l'organe à opérer à l'aide de la portion d'attrapage 30 qui joue un rôle de lacette. La lacette 30 est passée derrière l'organe à opérer 12, afin de rapprocher ce dernier de la zone dans laquelle le chirurgien va amener la pince à suture 22. Pendant la mise en place du système selon l'invention, l'opérateur, en général le chirurgien, veille à ce que la première partie de renfort 10A et la seconde partie de renfort 10B se situent en regard l'une de l'autre. Pour ce faire, il convient de passer la lacette 30 derrière la partie de l'organe 12 à opérer, puis d'amener en regard l'une de l'autre, les première et seconde parties de renfort 10A, 10B, à l'aide des moyens de préhension 28.

Dès lors, une pince à suture 22 peut être mise en place dans le système, en insérant chacune des deux branches 20 de la pince à suture 22 dans les logements 18A et 18B respectifs, comme illustré sur la figure 3.

Dans cette configuration, après insertion des branches 20 de la pince à suture 22 dans les logements 18A et 18B, les première et seconde parties de renfort 10A, 10B, en regard l'une de l'autre, se situent bien en face des zones actives 32 des branches 20, c'est-à-dire les zones dans lesquelles les moyens formant sutures sont formés. En l'espèce, la zone active 32 de l'une des deux branches 20 (en bas sur la figure 3) stocke les agrafes 26 avant utilisation, tandis que la zone active de l'autre des deux branches 20 comporte les moyens de repli des agrafes et la lame (en haut sur la figure 3).

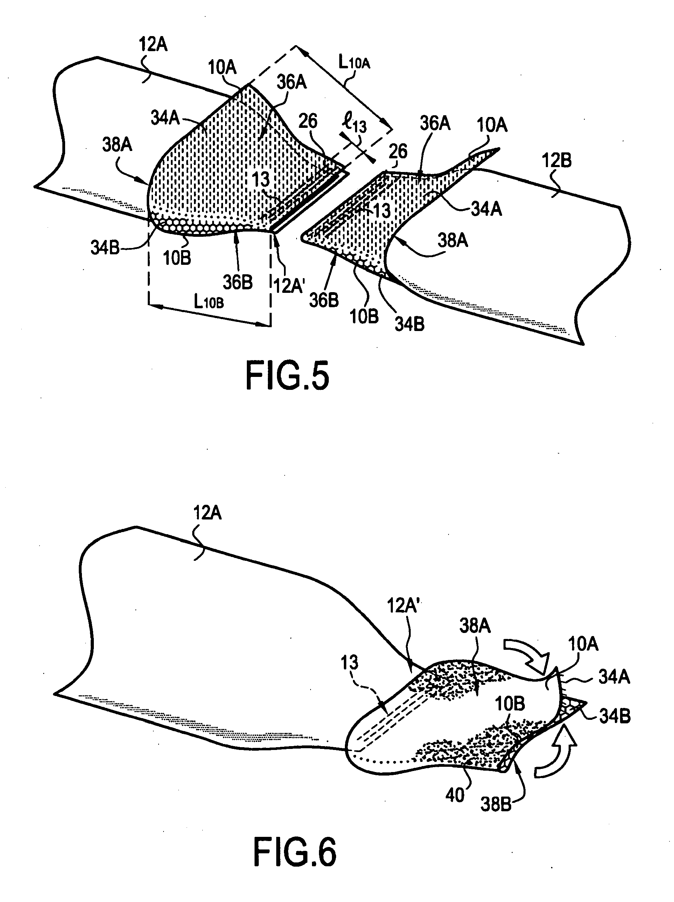

Au moment de l'utilisation de la pince à suture 22, les deux branches 20 sont resserrées l'une contre l'autre autour de l'organe 12 à opérer. Ce dernier est alors coupé en deux parties 12A et 12B qui sont simultanément suturées, en l'espèce agrafées à l'aide des agrafes 26 stockées dans les branches 20 de la pince à suture 22, comme illustré sur la figure 5. Lors de la section de l'organe 12, les première et seconde parties de renfort 10A et 10B, ainsi que la lacette 30, sont, elles aussi, coupées en deux, dans le sens longitudinal, parallèle au sens longitudinal des branches 20 de la pince à suture 22. Les parties 12A et 12B sont analogues, de sorte que la suite de la description n'est faite que pour la partie 12A.

Les fils d'assemblage 14 sont alors retirés de manière à séparer les moyens formant support 16 et les moyens de préhension 28, des première et seconde parties de renfort 10A, 10B, qui sont à présent assemblées à la partie 12A au niveau d'une suture 13, par l'intermédiaire des agrafes 26. Les moyens formant support 16 ainsi séparés peuvent être retirés. Les moyens de préhension 28 permettent de faciliter ce retrait.

La partie 12A' qui correspond à la partie 12A qui a été suturée et renforcée comporte donc une partie des agrafes 26 préalablement stockées dans la zone active 32 d'une des deux branches 20, et une portion des première et seconde parties de renfort 10A et 10B coupées en deux lanières.

Les première et seconde parties de renfort 10A et 10B sont alors repliées par dessus la zone suturée et renforcée 12A', comme illustré sur la figure 6, de manière à être assemblées l'une contre l'autre, en dehors de cette zone suturée et renforcée 12A'. A cet effet, les première et seconde parties de renfort 10A et 10B comportent des moyens formant maintien 34 aptes à coopérer pour maintenir ensemble les première et seconde parties de renfort 10A et 10B, l'une contre l'autre.

En l'espèce, les moyens formant maintien 34 comportent des premiers éléments de maintien 34A disposés sur la première partie de renfort 10A et des seconds éléments de maintien 34B disposés sur la seconde partie de renfort 10B.

Les premiers éléments de maintien 34A sont disposés sur une face 36A de la première partie de renfort 10A, qui, lorsque cette dernière était reliée aux moyens formant support 16, était tournée vers l'intérieur du logement 18A (voir figures 3 et 4) . En fait, cette face 36A est tournée vers les moyens formant sutures 26, avant la mise en place sur la zone 12 à sectionner et suturer de sorte de ne pas s'accrocher pendant la mise en place du système autour de l'organe. En outre, dans cette configuration, les moyens formant maintien 34 étant, au début de l'opération, tournés vers l'intérieur du logement 18A, ils ne peuvent pas jouer leur rôle à ce moment-là, puisqu'ils ne peuvent pas coopérer l'un avec l'autre.

Afin de faciliter la mise en place du système contre l'organe à opérer, une seconde face 38A (38B) de la première (deuxième) partie de renfort 10A (10B), qui est d'une part, opposée à la première face 36A (36B) qui comporte les éléments de maintien 34A, 34B, et d'autre part, tournée vers l'extérieur du logement 18A (18B), comporte préférentiellement un matériau antiadhérent, par exemple un matériau lubrifiant. En l'espèce, le matériau antiadhérent comporte un composé antiadhérent lubrifiant notamment de type gélatine 40 destiné à faciliter l'insertion du système auprès de l'organe 12 à opérer et à empêcher les adhérences anatomiques.

Après section et suture, comme illustré sur la figure 5, les premiers éléments de maintien 34A sont situés sur la face 36A opposée à la face 38A qui se trouve alors en regard de la zone suturée et renforcée 12A'.

Les faces respectives 36A et 36B des première et seconde parties de renfort 10A et 10B sont alors repliées l'une vers l'autre (voir les flèches sur la figure 6), de sorte que les premier et second éléments de maintien 34A et 34B puissent coopérer pour maintenir les première et seconde parties de renfort 10A et 10B l'une contre l'autre. A la fin de chaque mise en place, les faces opposées respectives 38A et 38B revêtues du composé antiadhérent 40, sont donc les faces visibles du système.

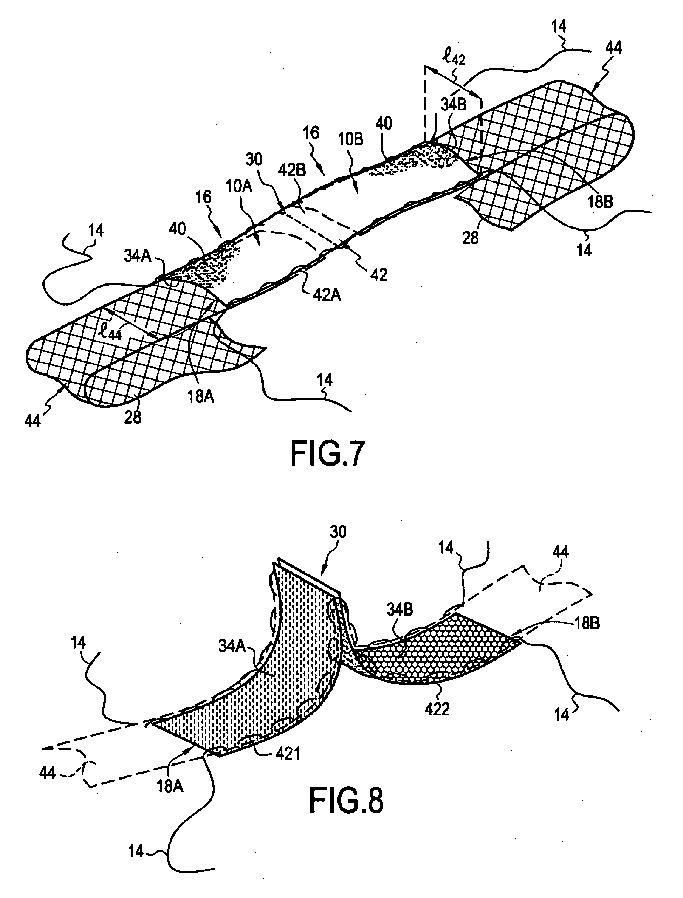

La figure 7 illustre un système formé de bandes assemblées. En l'espèce, les moyens de renfort 10 sont formés d'une première bande de tissu 42 en matériau biocompatible, en l'espèce en matériau biocompatible résorbable. Ce tissu présente une face 42B, qui comporte un matériau lubrifiant 40 tel que précité, et une face opposée 42A, qui comporte des moyens formant maintien 34A et 34B, tels que précités.

Pour des raisons de coût, le matériau lubrifiant et les moyens formant maintien peuvent être prévus uniquement dans les zones de tissu qui nécessitent leur présence. On comprend que les moyens formant maintien peuvent être uniquement prévus dans la zone de la bande 42 qui est destinée à être repliée telle qu'illustré sur la figure 6. En effet, il n'est pas nécessaire que la zone destinée à être agrafée par la suture 13, comporte, elle aussi, ces moyens formant maintien puisqu'ils ne joueront pas leur rôle.

Cette bande de tissu 42 forme à la fois les première et seconde parties de renfort 10A et 10B et la lacette 30. On comprend que les première et seconde parties de renfort 10A et 10B, et la lacette 30 pourraient aussi être formées de deux ou trois éléments distincts reliés ensemble. Dans ce dernier cas, les éléments pourraient être de matériaux différents, mais toujours en matériaux bio-résorbables.

Cette bande de tissu 42 est par ailleurs assemblée à chacune de ces deux extrémités, à deux bandes de tissu 44 respectives, en matériau biocompatible. Chacune de ces deux bandes de tissu 44 forme à la fois les moyens formant support 16 et les moyens formant préhension 28 précités.

Ces trois bandes de tissus 42, 44 et 44 sont reliées ensemble à l'aide des moyens d'assemblage 14, en l'espèce de deux fils 14 détachables qui sont disposés longitudinalement de part et d'autre de la largeur des bandes 42 et 44. Comme décrit précédemment, ces fils 14 sont d'une longueur telle qu'ils permettent leur préhension au voisinage des moyens de préhension 28 précités. Après opération, il convient simplement de tirer sur les deux extrémités respectives des deux fils 14 pour séparer les moyens formant renfort et les moyens formant support, et retirer ces derniers en les attrapant par les moyens de préhension 28.

On comprend que la taille des moyens formant renfort peut être adaptée à celle de la zone destinée à être suturée. En particulier, la largeur ℓ42 de la bande 42 peut être égale à celle de la largeur ℓ44 de la bande 44 ou bien être supérieure ou inférieure. Par ailleurs, la longueur de la bande 42 est choisie de manière qu'après section et suture, tel qu'illustré sur la figure 6, il reste suffisamment de tissu pour replier les première et seconde parties de renfort 10A et 10B, l'une contre l'autre, par dessus la suture 13. On pourra par exemple adapter la longueur de la bande 42 pour qu'après section et suture, les longueurs L10A et L10B (illustrées sur la figure 5) soient au moins égales à deux fois la largeur ℓ13 de la suture 13.

On veillera à ce que la largeur ℓ42 de la bande 42 et les longueurs L10A et L10B soient aussi choisies en fonction de la taille des branches 20 de la pince à suture 22 employée.

Selon la variante de réalisation de la figure 8, le système de mise en place de moyens formant renfort d'une suture est formée de quatre bandes : les deux bandes 44 du système de la figure 7 (en traits pointillés) et deux bandes 421 et 422 correspondant à la bande unique 42 de la figure 7 et formant les première et seconde parties de renfort 10A et 10B. Ces quatre bandes délimitent les deux logements 18A et 18B vers l'intérieur desquels sont tournées les faces des deux bandes 421 et 422 portant les premier et second éléments de maintien 34A et 34B. Dans le cas représenté sur la figure 8, les quatre bandes ne sont reliées entre elles que par deux fils 14 qui permettent à eux seuls de retenir le système et de former, à l'extrémité adjacente des bandes 421 et 422, la portion d'attrapage 30.

## Revendications

1. Système de mise en place de moyens formant renfort (10, 10A, 10B) d'une suture (13), comportant des moyens formant renfort (10, 10A, 10B) pour renforcer une zone (12) destinée à être suturée, comprenant une première partie de renfort (10A) et une deuxième partie de renfort (10B), une portion d'attrapage (30) reliant lesdites première et deuxième parties des moyens formant renfort (10, 10A, 10B) et permettant d'attraper la zone à suturer (12), et des moyens de passage (11, 18A, 18B) des deux branches (20) d'une pince à suture (22), tels que ladite première partie (10A) des moyens formant renfort (10, 10A, 10B) soit apte à être en regard de l'une des deux branches (20) et ladite seconde partie (10B) des moyens formant renfort (10, 10A, 10B) soit apte à être en regard de l'autre des deux branches (20), **caracterisé en ce que** ledit système de mise en place comporte en outre des moyens de préhension (28) formés par deux bandes reliées aux moyens formant renfort (10, 10A, 10B) qui s'étendent vers l'arrière en dehors de la zone des branches (20).

2. Système selon la revendication précédente, **caractérisé en ce que** lesdits moyens de passage sont deux ouvertures (11) de contour fermé situées respectivement sur lesdites première et deuxième parties des moyens formant renfort (10, 10A, 10B) et/ou les moyens de préhension (28); à l'écart de la portion d'attrapage (30).

3. Système selon la revendication 1 ou 2, **caractérisé en ce qu'**il comporte en outre des moyens formant support (16) et des moyens d'assemblage détachables (14) permettant d'assembler les moyens formant renfort (10, 10A, 10B) et les moyens formant support (16) pour délimiter lesdits moyens de passage formés de deux logements (18, 18A, 18B) destinés à loger les deux branches (20) d'une pince à suture (22), et **en ce qu'**avec les moyens formant support (16), la première partie de renfort (10A) forme l'un des deux logements (18A), tandis que la seconde partie de renfort (10B) forme l'autre (18B) des deux logements (18, 18A, 18B).

4. Système selon l'une quelconque des revendications précédentes, **caractérisé en ce qu'**il comporte des moyens formant maintien (34, 34A, 34B) permettant, après suture de la zone à suturer, de maintenir ensemble les première et deuxième parties de renfort (10, 10A, 10B).

5. Système selon la revendication précédente, **caractérisé en ce que** les moyens formant maintien (34, 34A, 34B) comportent des premiers éléments de maintien (34A) disposés sur la première partie de renfort (10A) et des seconds éléments de maintien (34B) disposés sur la seconde partie de renfort (10B), lesdits premiers et seconds éléments de maintien (34, 34A, 34B) étant aptes à coopérer pour maintenir lesdites première et seconde parties de renfort (10, 10A, 10B) l'une contre l'autre.

6. Système selon les revendications 3 et 5, **caractérisé en ce que** les moyens formant renfort (10, 10A, 10B) se présentent sous la forme d'une bande (10, 10A, 10B, 42) présentant une première face (36A, 36B, 42A) et une seconde face (38A, 38B, 42B), ladite première face (36A, 36B, 42A) étant tournée vers l'intérieur des logements (18A, 18B) respectifs lorsque les moyens formant support (16) et les moyens formant renfort (10, 10A, 10B) sont assemblés, **en ce que** les premier et second éléments de maintien (34, 34A, 34B) sont disposés sur ladite première face (36A, 36B, 42A), et **en ce qu'**après séparation des moyens formant support (16) et des moyens formant renfort (10, 10A, 10B), les première et seconde parties de renfort (10, 10A, 10B) sont aptes à être repliées sur elles-mêmes de sorte que les premier et second éléments de maintien (34, 34A, 34B) puissent s'engager les uns avec les autres pour maintenir lesdites première et seconde parties de renfort ensemble (10, 10A, 10B).

7. Système selon l'une quelconque des revendications 4 à 6, **caractérisé en ce que** la seconde face (38A, 38B, 42B), opposée à celle (36A, 36B, 42A) qui comporte les éléments de maintien (34, 34A, 34B), comporte un matériau antiadhérent.

8. Système selon la revendication précédente, **caractérisé en ce que** le matériau antiadhérent comporte un composé antiadhérent lubrifiant (40).

9. Système selon l'une quelconque des revendications précédentes, **caractérisé en ce que** lesdits moyens formant préhension (28) sont reliés aux moyens formant renfort (10A, 10B).

10. Système selon la revendication 3, **caractérisé en ce que** les moyens formant préhension (28) sont reliés aux moyens formant support (16).

11. Système selon la revendication 2 et l'une quelconque des revendications précédentes, **caractérisé en ce que** les deux logements (18, 18A, 18B) sont traversants.

12. Système selon l'une quelconque des revendications précédentes, **caractérisé en ce que** les moyens formant renfort (10, 10A, 10B) sont en matériau biocompatible.

13. Système selon la revendication précédente, **caractérisé en ce que** le matériau biocompatible est résorbable.

14. Système selon la revendication 3, **caractérisé en ce que** les moyens formant support (16) sont en matériau biocompatible.

## Claims

1. A placement system for putting reinforcement-forming means (10, 10A, 10B) for a suture (13) into place, comprising reinforcement-forming means (10, 10A, 10B) for reinforcing a zone (12) that is to be sutured, said means comprising a first reinforcement portion (10A) and a second reinforcement portion (10B), with a catching portion (30) interconnecting said first and second portions of the reinforcement-forming means (10, 10A, 10B) and serving to take hold of the zone (12) for suturing, and jaw-passing means (11, 18A, 18B) for passing two jaws (20) of a suture clamp (22), such that said first portion (10A) of the reinforcement-forming means (10, 10A, 10B) is suitable for facing one of the two jaws (20), and said second portion (10B) of the reinforcement-forming means (10, 10A, 10B) is suitable for facing the other one of the two jaws (20), said placement system being **characterized in that** it further comprises grip means (28) formed by two strips connected to the reinforcement-forming means (10, 10A, 10B) and extending rearwards away from the region of the jaws (20).

2. A system according to the preceding claim, **characterized in that** said jaw-passing means comprise two openings (11) of closed outline situated respectively in said first and second portions of the reinforcement-forming means (10, 10A, 10B) and/or the grip means (28), away from the catching portion (30).

3. A system according to claim 1 or claim 2, **characterized in that** it further comprises support-forming means (16) and detachable assembly means (14) enabling the reinforcement-forming means (10, 10A, 10B) and the support-forming means (16) to be assembled together to define said jaw-passing means that are formed by two housings (18, 18A, 18B) for housing the two jaws (20) of a suture clamp (22), and **in that** together with the support-forming means (16), the first reinforcement portion (10A) forms one of the two housings (18A), while the second reinforcement portion (10B) forms the other one (18B) of the two housings (18, 18A, 18B).

4. A system according to any preceding claim, **characterized in that** it includes holder-forming means (34, 34A, 34B) serving, after the zone for suturing has been sutured, to hold the first and second reinforcement portions (10, 10A, 10B) together.

5. A system according to the preceding claim, **characterized in that** the holder-forming means (34, 34A, 34B) have first holder elements (34A) disposed on the first reinforcement portion (10A) and second holder elements (34B) disposed on the second reinforcement portion (10B), said first and second holder elements (34, 34A, 34B) being suitable for co-operating to hold said first and second reinforcement portions (10, 10A, 10B) one against the other.

6. A system according to claims 3 and 5, **characterized in that the** reinforcement-forming means (10, 10A, 10B) are in the form of a strip (10, 10A, 10B, 42) having a first face (36A, 36B, 42A) and a second face (38A, 38B, 42B), said first face (36A, 36B, 42A) facing towards the inside of the respective housings (18A, 18B) when the support-forming means (16) and the reinforcement-forming means (10, 10A, 10B) are assembled together, **in that** the first and second holder means (34, 34A, 34B) are disposed on said first face (36A, 36B, 42A), and **in that**, after the support-forming means (16) and the reinforcement-forming means (10, 10A, 10B) have been separated, the first and second reinforcement portions (10, 10A, 10B) are suitable for being folded over in half so that the first and second holder elements (34, 34A, 34B) can engage mutually so as to hold said first and second reinforcement portions (10, 10A, 10B) together.

7. A system according to any one of claims 4 to 6, **characterized in that** the second face (38A, 38B, 42B) opposite from the face (36A, 36B, 42A) having the holder elements (34, 34A, 34B) includes a non-stick material.

8. A system according to the preceding claim, **characterized in that** the non-stick material comprises a lubricating non-stick compound (40).

9. A system according to any preceding claim, **characterized in that** said grip-forming means (28) are connected to the reinforcement-forming means (10A, 10B).

10. A system according to claim 3, **characterized in that** the grip-forming means (28) are connected to the support-forming means (16).

11. A system according to claim 2 and any preceding claim, **characterized in that** the two housings (18, 18A, 18B) are through housings.

12. A system according to any preceding claim, **characterized in that** the reinforcement-forming means (10, 10A, 10B) are made of biocompatible material.

13. A system according to the preceding claim, **characterized in that** the biocompatible material is resorbable.

14. A system according to claim 3, **characterized in that** the support-forming means (16) are made of biocompatible material.

## Patentansprüche

1. System zum Einsetzen von Mitteln (10, 10A, 10B), die eine Verstärkung einer Naht (13) bilden, umfassend Verstärkungsmittel (10, 10A, 10B) zum Verstärken eines zu nähenden Bereichs (12), die einen ersten Verstärkungsteil (10A) sowie einen zweiten Verstärkungsteil (10B) aufweisen, einen Erfassungsabschnitt (30), welcher den ersten und den zweiten Teil der Verstärkungsmittel (10, 10A, 10B) verbindet und ermöglicht, den zu nähenden Bereich (12) zu erfassen, sowie Durchgangsmittel (11, 18A, 18B) der beiden Schenkel (20) einer Nahtzange (22), welche derart sind, daß der erste Teil (10A) der Verstärkungsmittel (10, 10A, 10B) in der Lage ist, dem einem der beiden Schenkel (20) gegenüber zu liegen und der zweite Teil (10B) der Verstärkungsmittel (10, 10A, 10B) in der Lage ist, dem anderen der beiden Schenkel (20) gegenüber zu liegen, **dadurch gekennzeichnet, daß** das Einsetzsystem ferner Greifmittel (28) aufweist, die von zwei mit den Verstärkungsmitteln (10, 10A, 10B) verbundenen Streifen gebildet sind und die sich nach hinten außerhalb des Bereichs der Schenkel (20) erstrecken.

2. System nach dem vorhergehenden Anspruch, **dadurch gekennzeichnet, daß** die Durchgangsmittel zwei Öffnungen (11) mit geschlossener Kontur sind, die sich an dem ersten bzw. dem zweiten Teil der Verstärkungsmittel (10, 10A, 10B) und/oder den Greifmitteln (28), im Abstand von dem Erfassungsabschnitt (30) befinden.

3. System nach Anspruch 1 oder 2, **dadurch gekennzeichnet, daß** es ferner Tragmittel (16) sowie lösbare Verbindungsmittel (14) aufweist, die ermöglichen, die Verstärkungsmittel (10, 10A, 10B) und die Tragmittel (16) zu verbinden, um die Durchgangsmittel, welche von zwei Aufnahmen (18, 18A, 18B) gebildet sind, die zur Aufnahme der beiden Schenkel (20) einer Nahtzange (22) bestimmt sind, zu begrenzen, und daß mit den Tragmitteln (16) der erste Verstärkungsteil (10A) die eine (18A) der beiden Aufnahmen bildet, während der zweite Verstärkungsteil (10B) die andere (18B) der beiden Aufnahmen (18, 18A, 18B) bildet.

4. System nach irgendeinem der vorhergehenden Ansprüche, **dadurch gekennzeichnet, daß** es Haltemittel (34, 34A, 34B) umfaßt, die ermöglichen, nach dem Nähen des zu nähenden Bereichs den ersten und den zweiten Verstärkungsteil (10, 10A, 10B) zusammen zu halten.

5. System nach dem vorhergehenden Anspruch, **dadurch gekennzeichnet, daß** die Haltemittel (34, 34A, 34B) erste Halteelemente (34A), die am ersten Verstärkungsteil (10A) angeordnet sind, sowie zweite Halteelemente (34B), die an dem zweiten Verstärkungsteil (10B) angeordnet sind, umfassen, wobei die ersten und die zweiten Halteelemente (34, 34A, 34B) geeignet sind, zusammenzuwirken, um den ersten und den zweiten Verstärkungsteil (10, 10A, 10B) aneinander zu halten.

6. System nach den Ansprüchen 3 und 5, **dadurch gekennzeichnet, daß** die Verstärkungsmitteln (10, 10A, 10B) in Form eines Streifens (10, 10A, 10B, 42) vorliegen, der eine erste Seite (36A, 36B, 42A) sowie eine zweite Seite (38A, 38B, 42B) aufweist, wobei die erste Seite (36A, 36B, 42A) der Innenseite der jeweiligen Aufnahmen (18A, 18B) zugewandt ist, wenn die Tragmittel (16) und die Verstärkungsmittel (10, 10A, 10B) verbunden sind, daß das erste und das zweite Halteelement (34, 34A, 34B) an der ersten Seite (36A, 36B, 42A) angeordnet sind, und daß nach dem Trennen der Tragmittel (16) und der Verstärkungsmittel (10, 10A, 10B) der erste und der zweite Verstärkungsteil (10, 10A, 10B) geeignet sind, aufeinander umgeschlagen zu werden, so daß das erste und das zweite Halteelement (34, 34A, 34B) miteinander in Eingriff gelangen können, um den ersten und den zweiten Verstärkungsteil (10, 10A, 10B) zusammen zu halten.

7. System nach irgendeinem der Ansprüche 4 bis 6, **dadurch gekennzeichnet, daß** die zweite Seite (38A, 38B, 42B), die derjenigen (36A, 36B, 42A) gegenüberliegt, welche die Halteelemente (34, 34A, 34B) umfaßt, ein Antihaftmaterial aufweist.

8. System nach dem vorhergehenden Anspruch, **dadurch gekennzeichnet, daß** das Antihaftmaterial eine Antihaft-Gleitmittelzusammensetzung (40) umfaßt.

9. System nach irgendeinem der vorhergehenden Ansprüche, **dadurch gekennzeichnet, daß** die Greifmittel (28) mit den Verstärkungsmitteln (10A, 10B) verbunden sind.

10. System nach Anspruch 3, **dadurch gekennzeichnet, daß** die Greifmittel (28) mit den Tragmitteln (16) verbunden sind.

11. System nach Anspruch 2 und irgendeinem der vorhergehenden Ansprüche, **dadurch gekennzeichnet, daß** die beiden Aufnahmen (18, 18A, 18B) durchgehend sind.

12. System nach irgendeinem der vorhergehenden Ansprüche, **dadurch gekennzeichnet, daß** die Verstärkungsmittel (10, 10A, 10B) aus biokompatiblem Material bestehen.

13. System nach dem vorhergehenden Anspruch, **dadurch gekennzeichnet, daß** das biokompatible Material resorbierbar ist.

14. System nach Anspruch 3, **dadurch gekennzeichnet, daß** die Tragmittel (16) aus biokompatiblem Material bestehen.
